# EUROPEAN PATENT SPECIFICATION

(11) **EP 0 720 846 B1**
(45) Date of publication and mention of the grant of the patent: **27.03.2002**
(21) Application number: 95308271.6
(22) Date of filing: 17.11.1995
(51) Int. Cl.: A61K 7/48, A61K 7/50

(54) **Cold creams containing acyl lactylates**
Acyllaktylate enthaltende Kühlsalbe
Emulsions mélangées à froid contenant des lactylates d'acyle

(30) Priority: 03.01.1995 US 367658; 25.04.1995 US 427787
(43) Date of publication of application: 10.07.1996
(73) Proprietor: UNILEVER PLC, London EC4P 4BQ (GB); UNILEVER N.V., 3013 AL Rotterdam (NL)
(72) Inventor: Znaiden, Alexander Paul, Trumbull, Connecticut 06611 (US); Wivell, Susan Lorraine Ciotti, Madison, Connecticut 06443 (US); Kickertz, Virginia Rae, Stratford, Connecticut 06497 (US)
(74) Representative: Evans, Jacqueline Gail Victoria

(56) References cited:
- EP-A- 0 572 271
- EP-A- 0 573 253
- EP-A- 0 595 528
- EP-A- 0 747 041
- WO-A-88/06880
- WO-A-95/23838
- DE-A- 4 215 502
- DE-C- 4 215 501
- GB-A- 2 056 309
- US-A- 3 098 795
- US-A- 4 198 311
- US-A- 4 867 965
- FLICK, E.W.: 'Cosmetic and toiletry formulations, Vol.3', 1995, NOYES PUBLICATIONS, NEW JERSEY/US
- FLICK, E.W.: 'Cosmetic and toiletry formulations, Vol.1', 1989, NOYES PUBLICATIONS, NEW JERSEY/USA
- FALBE, J., REGITZ, M.: 'Römpp Chemie Lexikon', 1995, THIEME VERLAG, STUTTGART

## Description

### Field of the Invention

The invention concerns cold cream cosmetics with improved water rinsability and drier feel than traditional cold creams.

### The Related Art

Modern cleansing creams are based on the solvent action of mineral oil to remove through binding either grime or make-up from skin. Removal of pigments of rouge, lipstick and face powder is a daily problem for most women. Cleansing creams have proved the ideal agent to perform this function.

Historically cleansing creams evolved over a period of centuries. Galen, a Greek physician around the year 150, is reported to be the inventor of the first cold cream. Skin preparations of that period consisted of animal and vegetable fats and oils. Beeswax and olive oil were the prime ingredients. Galen conceived the idea of incorporating water into a molten mixture of beeswax and olive oils. In the resultant product, the emollient effect of oil was accelerated, and a pleasant cooling effect was obtained from evaporation of water. Unfortunately the process of manufacture was slow and laborious. Products were also unstable and subject to developing rancidity. In time, sweet almond oil replaced the olive oil of the older formulations. Borax was introduced to cut manufacturing time, and a whiter and more stable emulsion resulted. A cold cream can be classified as a form of cleansing cream but with a heavier body. There is even a monograph for "cold cream" in the USP which describes its components as wax, mineral oil, water and sodium borate.

New cleansing chemicals and formulations have been developed over the last several decades that have provided technical advantage over traditional cold cream. These new products have lured away customers who seek less oily and better water rinsability properties.

Accordingly, it is an object of the present invention to provide a cosmetic composition with a less heavy, less greasy skinfeel than traditional cold creams.

It is another object of the present invention to provide a cosmetic composition having better water rinsability than that of traditional cold cream.

Still a further object of the present invention is to provide a cosmetic composition in emulsion form having stability no less than that of traditional cold cream even though containing potentially destabilizing additives such as α-hydroxy acids or their precursors.

These and other objects of the present invention will become more apparent through the following summary and detailed description.

### SUMMARY OF THE INVENTION

A cold cream cosmetic composition is provided that includes:
(i) from about 10 to about 50% by weight of water;
(ii) from about 5 to about 40% by weight of a wax;
(iii) from about 10 to about 80% by weight of mineral oil; and
(iv) from about 0.01 to about 20% by weight of a behenoyl lactylate salt.

### DETAILED DESCRIPTION OF THE INVENTION

Now it has been discovered that lactylates can substantially improve cold cream formulations. Lactylates provide products having a richer, creamier feel. On the skin, the product is observed to be less oily or drier, but very smooth and soft. Furthermore, spontaneous emulsification with water was observed with the cold creams containing lactylates. This was unusual in that cold cream, being less than 50% aqueous, normally does not blend well with water and forms a greasy film on the skin. Soap with warm water or an alcoholic astringent is usually needed to remove traditional cold cream residues left behind after tissuing. Typical surfactant materials other than lactylates were found to impart a blooming or rinsing effect not compatible with these wax-containing products.

Accordingly, an essential element of the present invention is that of a lactylate salt, especially sodium behenoyl lactylate. Other suitable cations to form the lactylate salt are the alkali metal, alkaline earth metal, ammonium and C₂-C₁₂ alkanolammonium cations. Lactylate salts are commercially available from the C.J. Patterson Division of RITA Corporation. Amounts of the lactylate salts will range from about 0.01 to about 20%, preferably from about 0.5 to about 10%, optimally from about 2 to about 5% by weight.

Mineral oil is another important element in the cosmetic compositions of the present invention. Amounts of mineral oil may range from about 10 to about 80%, preferably from about 25 to about 60%, optimally from about 40 to about 50% by weight.

Wax is yet another important element of compositions according to the present invention. Among suitable waxes are beeswax, ceresin, spermaceti, camauba, ozokerite, candelilla and mixtures of these waxes. Particularly preferred are combinations of beeswax and ceresin in weight ratios of about 5:1 to about 1:5, preferably about 2:1 to about 1:1. Amounts of total wax may range from about 0.5 to about 40%, preferably from about 1 to about 20%, optimally from about 2 to about 10% by weight.

Water is a further important element of compositions according to the present invention. Amounts of water may range from about 10 to about 50%, preferably from about 15 to about 35%, optimally from about 20 to about 30% by weight.

An alkali agent can optionally be incorporated as an element of cosmetic compositions according to the present invention. Illustrative alkali agents are C₂-C₁₀ alkanolamines (e.g. triethanolamine), sodium hydroxide, potassium hydroxide, ammonium hydroxide and borate salts. Sodium borate is most preferred, being available as borax in various states of hydration including anhydrous borax, borax decahydrate and borax pentahydrate. Amounts of the alkali agent may range from 0.01 to about 5%, preferably from about 0.5 to about 3%, optimally from about 1 to about 2% by weight.

Thickeners/viscosifiers may optionally be found in compositions to the present invention. Suitable thickeners include xanthan gum, sodium carboxymethyl cellulose, hydroxyalkyl and alkyl cellulose, and cross-linked acrylic acid polymers such as those sold by B.F. Goodrich under the Carbopol trademark (Carbomer, according to CTFA nomenclature). Most preferred is Carbopol 934®. Amounts of the thickeners/viscosifiers may range from about 0.01 to about 10%, preferably from about 0.1 to about 1% by weight.

Preservatives can also be incorporated in amounts effective to protect against growth of potentially harmful microorganism in cosmetic compositions according to the present invention. Levels of such preservatives may range from about 0.001 to about 1% by weight. Illustrative preservatives are methyl paraben, propyl paraben, imidazolidinyl urea, sodium dehydroxyacetate and benzyl alcohol. Other minor adjunct ingredients may also be included such as fragrances, opacifiers and colorants, each in their effective amounts to accomplish their respective functions.

The following examples will more fully illustrate the embodiments of this invention. All parts, percentages and proportions referred to herein and in the appended claims are by weight unless otherwise indicated.

### EXAMPLE 1

A clinical study was conducted among a group of twenty panelists selected as being non-cold cream users. These panelists were provided with a commercial cold cream (Formula A) and the same commercial product including 4% sodium behenoyl lactylate (Formula B). These formulas are outlined in Table I.

**TABLE I**

| **INGREDIENT** | **FORMULA (WT. %)** | |
|---|---|---|
| | **A** | **B** |
| Mineral Oil | 47.2 | 47.2 |
| Beeswax | 9.3 | 7.3 |
| Ceresin Wax | 6.0 | 4.0 |
| Sodium Behenoyl Lactylate | 0 | 4.0 |
| Carbopol 934® (2% Aqueous) | 10.0 | 10.0 |
| Borax | 1.7 | 1.7 |
| Fragrance | 0.2 | 0.2 |
| Water | to 100 | to 100 |

After the panelists used both products, they were requested to compare Formulas A and B according to the following attributes on a preference basis.

**TABLE II**

| **PANEL TEST RESULTS** | | | |
|---|---|---|---|
| **ATTRIBUTE** | **FORMULA A PREFERRED** | **FORMULA B PREFERRED** | **NO PREFERENCE** |
| Being Easy To Use | 4 | 8 | 8 |
| Product Goes On Smoothly | 12 | 4 | 4 |
| Removing Dirt & Oils From Skin | 4 | 12 | 4 |
| Being An Effective Cleanser | 4 | 12 | 4 |
| Removing Eye Make-Up Easily | 4 | 12 | 4 |
| Removing Facial Make-Up Easily | 4 | 12 | 4 |
| Removing Eye Make-Up Completely | 4 | 12 | 4 |
| Feeling Light On The Skin | 8 | 4 | 8 |
| Non-Irritating To Skin | 8 | 8 | 4 |
| Non-Irritating To Eyes | 8 | 8 | 4 |
| Leaving Skin Feeling Soft/Smooth | 4 | 12 | 4 |
| Gentle Enough To Use Around Eyes | 4 | 8 | 8 |
| Rinsing Well With Water | 4 | 8 | 8 |
| Having A Pleasant Scent | 8 | 4 | 8 |
| Deep Cleaning Pores | 4 | 12 | 4 |
| Wiping Off Easily | 8 | 4 | 8 |
| Not Leaving Skin Feeling Dry | 8 | 8 | 4 |
| No Greasy Residue | 4 | 8 | 8 |
| No Oily Residue | 4 | 8 | 8 |
| Leaving Skin Feeling Clean | 4 | 8 | 8 |
| Cleansing Quickly | 4 | 8 | 8 |

From Table II it is seen that Formula B with behenoyl lactylate is strongly preferred in several categories, and only a few categories is it inferior to the traditional cold cream Formula A. The highly preferred categories were that of removing dirt/oils, effective cleanser, removing eye make-up easily, removing facial make-up easily, removing eye make-up completely, leaving skin feeling soft/smooth and deep cleaning of pores.

### EXAMPLE 2

A set of formulations were prepared to evaluate performance of different chain length acyl lactylates. The following base cold cream was prepared and various lactylates were incorporated therein at 4% by weight. These were compared to a control without lactylate but having 2% more of both beeswax and ceresin wax than the base cold cream formula.

**TABLE III**

| Base Cold Cream Formula | |
|---|---|
| **INGREDIENT** | **WEIGHT %** |
| Mineral Oil | 47.2 |
| Beeswax | 7.3 |
| Ceresin Wax | 4.0 |
| Lactylate Salt | 4.0 |
| Carbopol 934® (2% Aqueous) | 10.0 |
| Borax | 1.7 |
| Fragrance | 0.2 |
| Water | to 100 |

**TABLE IV**

| Stability Profile and Physical Properties | | | | | |
|---|---|---|---|---|---|
| **PRODUCT** | **RT/22°C** | | **37°C** | **43°C** | **50°C** |
| No Lactylate (Control) | form skinfeel | cream greasy, not rinsable | same same | same same | same same |
| Behenoyl Lactylate (C22) | form skinfeel | cream (harder) less greasy, rinsable | same same | same same | same same |
| Isostearoyl Lactylate (C18) | form skinfeel | cream (soft) greasy, not rinsable | same same | same same | significant phase separation after 1 mo. |
| Lauroyl Lactylate (C12) | form skinfeel | thin lotion greasy, not rinsable | n/a n/a | n/a n/a | n/a n/a |

From the results in Table IV it is evident that only the behenoyl lactylate formulation is rinsable in contrast to the control and to those with isostearoyl and lauroyl lactylate. Furthermore, the behenoyl lactylate formulation is less greasy than all of the others tested and has a hardness much closer to the control formula than the formulations incorporating any of the other lactylates.

### EXAMPLE 3

A further formulation of a borax-free cold cream is hereunder illustrated. The formulas are outlined in Table V.

| **INGREDIENT** | **FORMULA (WT. %)** | | | |
|---|---|---|---|---|
| | **G** | **E** | **F** | **H** |
| Mineral Oil | 35.2 | 46.0 | 48.2 | 49.0 |
| Sodium Behenoyl Lactylate | 6.0 | 6.0 | 4.0 | 5.0 |
| Beeswax | 3.5 | 2.0 | 1.0 | 2.0 |
| Ceresin Wax | 2.0 | 1.5 | 0.9 | 2.0 |
| Carbopol 934 (2% Aqueous) | 5.0 | 5.0 | 5.0 | 5.0 |
| Butylene Glycol | 3.0 | 4.0 | 4.0 | 4.5 |
| PEG-100 Stearate | 1.5 | 1.5 | 1.8 | 1.8 |
| Petrolatum (2.5 Soft) | 1.0 | 1.0 | 1.0 | 1.0 |
| Glycerin | 0.9 | 1.3 | 1.0 | 1.0 |
| Sorbitan Sesquioleate | 0.9 | 0.8 | 1.0 | 0.9 |
| Glyceral Stearate | 0.5 | 0.7 | 1.0 | 0.5 |
| Cetyl Alcohol | 0.3 | 0.3 | 0.3 | 0.3 |
| Stearic Acid | 0.3 | 0.3 | 0.3 | 0.3 |
| Fragrance | 0.2 | 0.2 | 0.2 | 0.2 |
| Dimethicone | 0.1 | 0.1 | 0.1 | 0.1 |
| Triethanolamine | 0.1 | 0.1 | 0.1 | 0.1 |
| Glydant Plus® | 0.1 | 0.1 | 0.1 | 0.1 |
| Glycolic Acid | 0.02 | 0.02 | 0.02 | 0.02 |
| Vitamin E Acetate | 0.01 | 0.01 | 0.01 | 0.01 |
| Ascorbyl Palmitate | 0.01 | 0.01 | 0.01 | 0.01 |
| Hydroxycaprylic Acid | 0.01 | 0.01 | 0.01 | 0.01 |
| Water | to 100 | to 100 | to 100 | to 100 |

The foregoing description and Examples illustrate selected embodiments of the present invention.

## Claims

1. A cold cream cosmetic composition comprising:
(i) from about 10 to about 50% by weight of water;
(ii) from about 0.5 to about 40% by weight of a wax;
(iii) from about 10 to about 80% by weight of mineral oil;
(iv) from about 0.01 to about 20% by weight of a behenoyl lactylate salt.

2. A composition according to claim 1 wherein the behenoyl lactylate is present in an amount from about 0.5 to about 10% by weight.

3. A composition according to claim 1 or claim 2 further comprising an alkali agent selected from C₂-C₁₀ alkanolamine, sodium hydroxide, potassium hydroxide or borate salts.

4. A composition according to claim 3 wherein the alkali agent is a borate salt.

5. A composition according to any of the preceding claims wherein the mineral oil is present in an amount from about 25 to about 60% by weight.

6. A composition according to any of the preceding claims wherein the wax is selected from beeswax, ceresin, spermaceti, carnauba, ozokerite, candelilla and mixtures thereof.

7. A composition according to claim 6 wherein the wax is a mixture of beeswax and ceresin wax in a weight ratio of about 5:1 to about 1:5.

## Patentansprüche

1. Kosmetische Kühlsalbenzusammensetzung, umfassend:
(i) etwa 10 bis etwa 50 Gewichtsprozent Wasser;
(ii) etwa 0,5 bis etwa 40 Gewichtsprozent eines Wachses;
(iii) etwa 10 bis etwa 80 Gewichtsprozent Mineralöl;
(iv) etwa 0,01 bis etwa 20 Gewichtsprozent eines Behenoyllactylatsalzes.

2. Zusammensetzung nach Anspruch 1, worin das Behenoyllactylat in einer Menge von etwa 0,5 bis etwa 10 Gewichtsprozent vorliegt.

3. Zusammensetzung nach Anspruch 1 oder Anspruch 2, die weiterhin ein alkalisches Mittel umfasst, ausgewählt aus C₂-C₁₀-Alkanolamin, Natriumhydroxid, Kaliumhydroxid oder Boratsalzen.

4. Zusammensetzung nach Anspruch 3, worin das alkalische Mittel ein Boratsalz ist.

5. Zusammensetzung nach einem der vorangehenden Ansprüche, worin das Mineralöl in einer Menge von etwa 25 bis etwa 60 Gewichtsprozent vorliegt.

6. Zusammensetzung nach einem der vorangehenden Ansprüche, worin das Wachs aus Bienenwachs, Ceresin, Spermaceti, Carnauba, Ozokerit, Candelilla und Gemischen davon ausgewählt ist.

7. Zusammensetzung nach Anspruch 6, worin das Wachs ein Gemisch von Bienenwachs und Ceresinwachs in einem Gewichtsverhältnis von etwa 5:1 bis etwa 1:5 ist.

## Revendications

1. Composition cosmétique sous forme d'une crème émulsionnée à froid comprenant :
(i) d'environ 10 % à environ 50 % en poids d'eau ;
(ii) d'environ 5 % à environ 40 % en poids d'une cire ;
(iii) d'environ 10 à environ 80 % en poids d'huile minérale ; et
(iv) d'environ 0,01 à environ 20 % en poids d'un sel de béhénoyl lactylate.

2. Composition selon la revendication 1, dans laquelle le béhénoyl lactylate est présent dans une quantité allant d'environ 0,5 à environ 10 % en poids.

3. Composition selon la revendication 2 ou la revendication 3, comprenant en outre un sel d'alcanolamine en C₂ - C₁₀, d'hydroxyde de sodium, d'hydroxyde de potassium ou de borate.

4. Composition selon la revendication 3, dans laquelle l'agent alcali est un sel de borate.

5. Composition selon l'une quelconque des revendications précédentes, dans laquelle l'huile minérale est présente dans une quantité allant d'environ 25 à environ 60 % en poids.

6. Composition selon ;l'une quelconque des revendications précédentes, dans laquelle la cire est sélectionnée à partir de la cire d'abeille, de la cérésine, du spermaceti, de la cire de carnauba, de l'ozokérite, de la cire de candélilla et de mélanges de ceux-ci.

7. Composition selon la revendication 6, dans laquelle la cire est un mélange de cire d'abeille et de cire de cérésine dans un rapport en poids d'environ 5 pour 1 à 1 pour 5.
